# EUROPEAN PATENT SPECIFICATION

(11) **EP 2 153 770 B1**
(45) Date of publication and mention of the grant of the patent: **26.10.2011**
(21) Application number: 09010409.2
(22) Date of filing: 12.08.2009
(51) Int. Cl.: A61B 1/00, A61B 5/00

(54) **Optical probe and three-dimensional image acquisition apparatus**
Optische Sonde und dreidimensionale Bilderwerbsvorrichtung
Sonde optique et appareil d'acquisition d'images tridimensionnelles

(30) Priority: 15.08.2008 JP 2008209193
(43) Date of publication of application: 17.02.2010
(73) Proprietor: FUJIFILM Corporation, Tokyo 106-8620 (JP)
(72) Inventor: Watanabe, Daisuke, Ashigarakami-gun Kanagawa 258-8538 (JP)
(74) Representative: Höhfeld, Jochen

(56) References cited:
- EP-A- 1 935 356
- US-A1- 2003 004 412

## Description

### BACKGROUND OF THE INVENTION

### Field of Invention

The present invention relates to a three-dimensional image acquisition apparatus, and more particularly, to a three-dimensional image acquisition apparatus capable of acquiring accurate three-dimensional volume data even when there occur mechanical operation irregularities during scanning at a measurement position.

### Description of the Related Art

Conventionally, as an endoscope apparatus for observing inside of a body cavity of a living body, an electronic endoscope apparatus which emits illumination light inside the body cavity of the living body, picks up an image using reflected light, and displays the image on a monitor or the like has been widely used, and has been utilized in various fields. Most of endoscope apparatuses have a forceps port. A probe introduced into the body cavity via the forceps port can be used to perform biopsy and treatment of tissue inside the body cavity.

Meanwhile, development of a tomographic image acquisition apparatus which acquires a tomographic image of a living body or the like without cutting an object to be measured such as living tissue has been recently advanced. For example, as described in Japanese Patent Application Laid-Open No. 2002-148185, there is known an optical tomographic imaging apparatus which utilizes an optical coherence tomography (OCT) technique by use of interference of low-coherence light. The optical tomographic imaging apparatus using the OCT technique divides the low-coherence light emitted from a light source composed of a super luminescent diode (SLD) or the like into signal light and reference light, slightly shifts a frequency of the reference light or the signal light by a piezoelectric element or the like, causes the reference light to interfere with a returning light which is obtained by allowing the signal light to enter a portion to be measured and be reflected at a predetermined depth of the portion to be measured, and measures a light intensity of an interference light by heterodyne detection, thereby acquiring tomographic information. With the optical tomographic imaging apparatus, by moving a little a movable mirror or the like arranged on the optical path of the reference light and thereby slightly changing the optical path length of the reference light the optical path length of the reference light corresponds to the optical path length of the signal light, and information at the depth of the portion to be measured can be obtained. Also, optical tomographic images can be acquired over a predetermined scanning area by repeating the measurement with the incident point of the signal light being displaced little by little. Furthermore, volume data for a three-dimensional image can be acquired by displacing the incident point of the signal light in a direction perpendicular to a tomographic plane and acquiring a plurality of optical tomographic images.

Such OCT apparatus (the optical tomographic imaging apparatus) enables an operator to observe the portion to be measured finely (a resolution limit of about 10 µm). For example, invasion depth diagnosis of early cancers is enabled by inserting an OCT probe (an optical probe) into the forceps port of the endoscope apparatus, guiding the signal light and the returning light of the signal light from the living body, and acquiring the optical tomographic images inside the body cavity.

US 2003/004412 A1 discloses a three-dimensional image acquistion apparatus in accordance with the preamble of claim 1.

### SUMMARY OF THE INVENTION

The three-dimensional image is generated from the volume data as described above on the assumption that the adjacent tomographic images in the volume data are respectively acquired at regular intervals. However, there is a problem that the acquisition intervals of the tomographic images become irregular and accurate three-dimensional volume data cannot be generated when there occur operational irregularities in a mechanism for scanning the incident point of the signal light.

The present invention has been made in view of such circumstances, and it is an object of the present invention to provide a three-dimensional image acquisition apparatus capable of acquiring accurate three-dimensional volume data even when there occur mechanical operation irregularities during scanning at a measurement position. The present invention provides a three-dimensional image acquisition apparatus having the features of claim 1. Preferred embodiments are defined by the dependent claims.

In the present invention, the first markers are provided at predetermined intervals on the distal end portion of the sheath along the longitudinal direction of the sheath. Therefore, by extracting the first markers from a plurality of acquired optical tomographic images and adjusting a number of the optical tomographic images such that the number of optical tomographic images between the optical tomographic images from which the first markers are extracted is equal to each other, accurate three-dimensional volume data can be acquired even when there occur mechanical operation irregularities during scanning at a measurement position.

Preferably, in the optical probe according to the first aspect, the first markers are provided on an inner wall surface of the distal end portion of the sheath.

Accordingly, the first markers can be extracted from the plurality of optical tomographic images which are acquired appropriately.

Preferably, in the optical probe according to the first or second aspect, a second marker which is a straight line parallel to the longitudinal direction of the sheath is provided on the distal end portion of the sheath.

By extracting the second marker provided as described above and rotating the plurality of optical tomographic images such that the extracted second marker is aligned with each other, accurate three-dimensional volume data can be acquired even when there occur mechanical operation irregularities during scanning at the measurement position.

Preferably, in the optical probe, the second marker is provided on the inner wall surface of the distal end portion of the sheath.

Accordingly, the second marker can be extracted from the plurality of optical tomographic images which are acquired appropriately.

Preferably, in the optical probe, the first markers and the second marker are provided perpendicular to each other.

Accordingly, the first markers and the second marker can be provided so as not to prevent the acquisition of the optical tomographic image.

Accordingly, accurate three-dimensional volume data can be acquired even when mechanical operation irregularities during scanning at a measurement position occur.

According to claim 2, accurate three-dimensional volume data can be acquired even when mechanical operation irregularities during scanning at a measurement position occur.

Preferably, the three-dimensional image acquisition apparatus further includes a generation device for generating a three-dimensional image based on the plurality of optical tomographic images.

Accordingly, an accurate three-dimensional image can be generated even when mechanical operation irregularities during scanning at a measurement position occur.

With the present invention, the first markers are provided at predetermined intervals on the distal end portion of the sheath along the longitudinal direction of the sheath. Therefore, by extracting the first markers from the plurality of acquired optical tomographic images and adjusting the number of optical tomographic images such that the number of optical tomographic images between the optical tomographic images from which the first markers are extracted is equal to each other, accurate three-dimensional volume data can be acquired even when there occur mechanical operation irregularities during scanning at a measurement position.

### BRIEF DESCRIPTION OF THE DRAWINGS

Fig. 1 is an external view illustrating a diagnostic imaging apparatus 10 according to the present invention;
Fig. 2 is a block diagram illustrating an internal configuration of an OCT processor 400 and an OCT probe 600;
Fig. 3 is a sectional view of the OCT probe 600;
Fig. 4 is a view illustrating a state in which a tomographic image is acquired by using the OCT probe 600 pulled out of a forceps port 156 of an endoscope 100;
Figs. 5A and 5B are views illustrating volume data and a three-dimensional image generated based on the volume data, respectively;
Fig. 6 is a view of a probe outer tube 620 according to a present embodiment; Fig: 7 is a flowchart illustrating generation of three-dimensional volume data by using the probe outer tube 620 with markers;
Figs. 8A and 8B are views illustrating optical tomographic images acquired by using the probe outer tube 620 with markers;
Fig. 9 is a view illustrating that marker portions 648 are brought in phase with each other by rotating each optical tomographic image;
Fig. 10 is a view illustrating a state in which an image 640 is extracted; and Figs. 11A and 11B are views illustrating an example of a movement irregularity correction marker 630, and the acquisition positions of the image 640 and an image 642.

### DETAILED DESCRIPTION OF THE PREFERRED EMBODIMENTS

In the following, a preferred embodiment for carrying out the present invention will be described.

### <Appearance of diagnostic imaging apparatus>

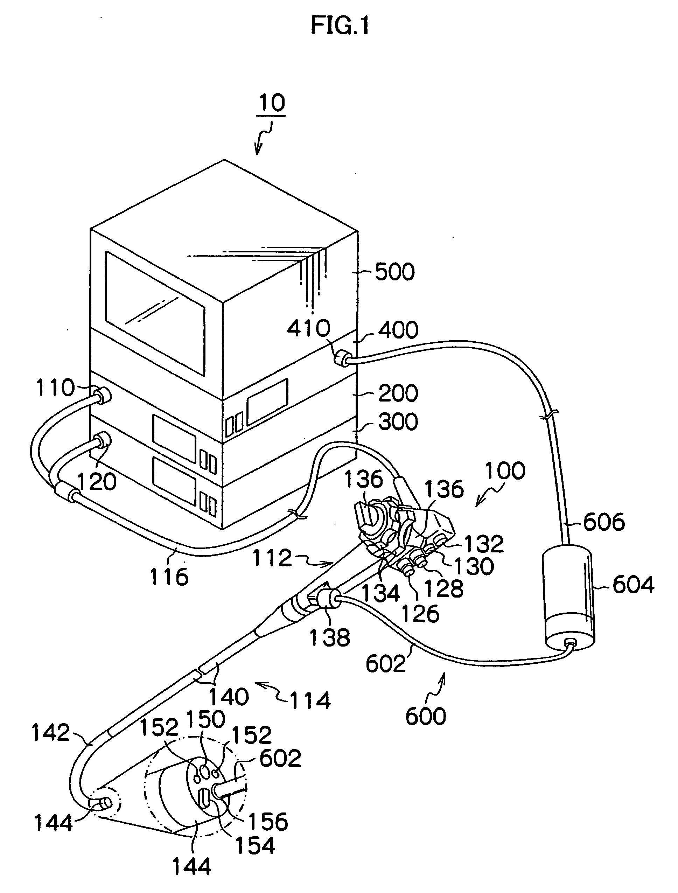

Fig. 1 is an external view illustrating a diagnostic imaging apparatus 10 according to the present invention.

As shown in Fig. 1, the diagnostic imaging apparatus 10 mainly includes an endoscope 100, an endoscope processor 200, a light source device 300, an OCT processor 400, and a monitor device 500. The endoscope processor 200 may incorporate the light source device 300.

The endoscope 100 includes a hand-side operation portion 112 and an insertion portion 114 which is provided next to the hand-side operation portion 112. An operator grasps the hand-side operation portion 112 to operate the endoscope 100, and inserts the insertion portion 114 into the body of a subject to observe the inside of the body.

The hand-side operation portion 112 includes a forceps insertion portion 138. The forceps insertion portion 138 communicates with a forceps port 156 of a distal end portion 144. In the diagnostic imaging apparatus 10 according to the present invention, an OCT probe 600 is inserted from the forceps insertion portion 138 and is pulled out of the forceps port 156. The OCT probe 600 includes an insertion portion 602 which is inserted from the forceps insertion portion 138 and is pulled out of the forceps port 156, an operation portion 604 with which an operator operates the OCT probe 600, and a cable 606 which is connected to the OCT processor 400 via a connector 410.

### <Configuration of endoscope, endoscope processor, and light source device>

### [Endoscope]

An observation optical system 150, an illumination optical system 152, and a CCD (not illustrated) are provided in the distal end portion 144 of the endoscope 100.

The observation optical system 150 forms an image of a subject (a subject image) on a light receiving surface of the unillustrated CCD. The CCD converts the subject image formed on the light receiving surface to an electrical signal by each light receiving element. The CCD in a present embodiment is a color CCD in which color filters having three primary colors: red (R), green (G), and blue (B), are respectively arranged on one pixel in a predetermined array (a Bayer array, a honeycomb array).

### [Light source device]

The light source device 300 causes visible light to enter an unillustrated light guide. One end of the light guide is connected to the light source device 300 via an LG connector 120, and the other end of the light guide faces the illumination optical system 152. The light emitted from the light source device 300 is emitted from the illumination optical system 152 through the light guide, to illuminate the visual field area of the observation optical system 150.

### [Endoscope processor]

An image signal (an analog image signal) output from the CCD is input to the endoscope processor 200 via an electrical connector 110. The analog image signal is converted to a digital image signal in the endoscope processor 200, and a processing required for displaying the image signal on the screen of the monitor device 500 is performed.

An image signal representing a tomographic image output from the OCT processor 400 is also input to the endoscope processor 200. The endoscope processor 200 generates three-dimensional volume data based on a plurality of tomographic image signals. A required process is also performed on the generated three-dimensional image from the three-dimensional volume data by the endoscope processor 200, and the three-dimensional image is output to the monitor device 500.

As described above, the data of the observed image acquired by the endoscope 100 is output to the endoscope processor 200, and the image is displayed on the monitor device 500 connected to the endoscope processor 200.

### <Internal configuration of OCT processor and OCT probe>

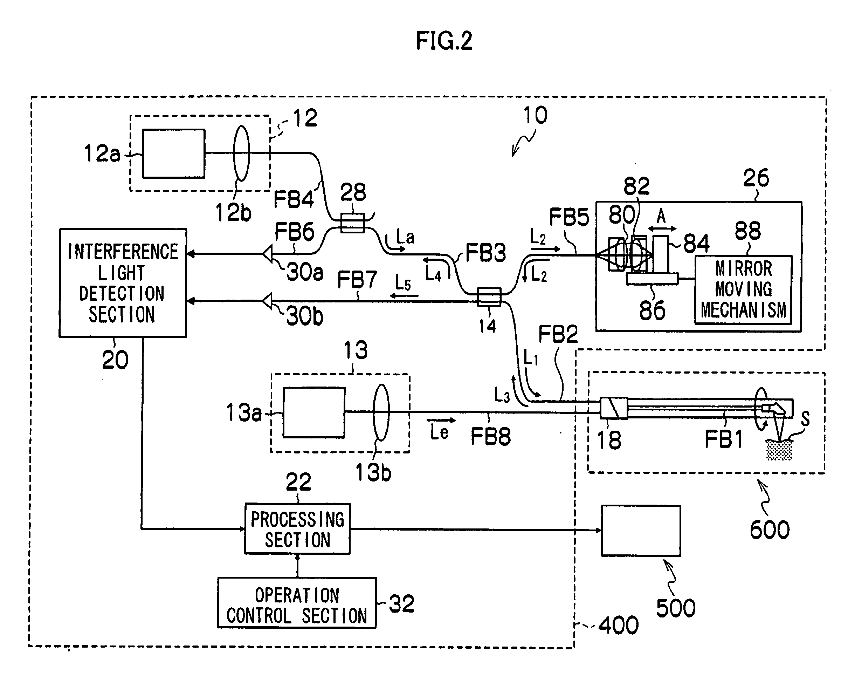

Fig. 2 is a block diagram illustrating an internal configuration of the OCT processor 400 and the OCT probe 600.

### [OCT processor]

The OCT processor 400 and the OCT probe 600 shown in Fig. 2 are used for acquiring the optical tomographic image of an object to be measured by using the optical coherence tomography (OCT) technique. The OCT processor 400 and the OCT probe 600 include a first light source (a first light source unit) 12 which emits light La for measurement, an optical fiber coupler (a branching/combining section) 14 which branches the light La emitted from the first light source 12 into measurement light (a first light flux) L1 and reference light L2, and combines returning light L3 from the object to be measured S as a subject and the reference light L2, thereby generating interference light L4, an optical probe 16 including a rotation-side optical fiber FB1 which guides the measurement light L1 branched by the optical fiber coupler 14 to the object to be measured and guides the returning light L3 from the object to be measured, a fixed-side optical fiber FB2 which guides the measurement light L1 to the rotation-side optical fiber FB1 and guides the returning light L3 guided by the rotation-side optical fiber FB1, an optical connector 18 via which the rotation-side optical fiber FB1 and the fixed-side optical fiber FB2 are rotatably connected, the optical connector 18 transmitting the measurement light L1 and the returning light L3, an interference light detection section 20 which detects the interference light L4 generated by the optical fiber coupler 14 as an interference signal, and a processing section 22 which processes the interference signal detected by the interference light detection section 20 to acquire the optical tomographic image (also referred to simply as "tomographic image" below). The optical tomographic image acquired by the processing section 22 is displayed on the monitor device 500.

The OCT processor 400 also includes a second light source (a second light source unit) 13 which emits aiming light (a second light flux) Le for indicating a mark for measurement, an optical path length adjustment section 26 which adjusts an optical path length of the reference light L2, an optical fiber coupler 28 which branches the light La emitted from the first light source 12, detection sections 30a and 30b which detect the interference light L4 and L5 combined by the optical fiber coupler 14, and an operation control section 32 which inputs various conditions to the processing section 22 and changes settings, etc. thereof.

In the OCT processor 400 shown in Fig. 2, various optical fibers FB (FB3, FB4, FB5, FB6, FB7, FB8 and the like) including the rotation-side optical fiber FB1 and the fixed-side optical fiber FB2 are used as an optical path for guiding and transmitting various light including the emission light La, the aiming light Le, the measurement light L1, the reference light L2 and the returning light L3 between components such as each optical device.

The first light source 12 emits the light for OCT measurement (for example, laser light or low-coherence light having a wavelength of 1.3 µm). The first light source 12 includes a light source 12a which emits the laser light or low-coherence light La, and a lens 12b which collects the light La emitted from the light source 12a. Although described in detail later, the light La emitted from the first light source 12 is divided into the measurement light L1 and the reference light L2 by the optical fiber coupler 14 via the optical fibers FB4 and FB3, and the measurement light L1 is input to the optical connector 18.

The second light source 13 emits visible light as the aiming light Le for allowing a portion to be measured to be easily confirmed. For example, red semiconductor laser light having a wavelength of 0.66 µm, He-Ne laser light having a wavelength of 0.63 µm, and blue semiconductor laser light having a wavelength of 0.405 µm can be used. The second light source 13 includes a semiconductor laser 13a which emits red, blue or green laser light, for example, and a lens 13b which collects the aiming light Le emitted from the semiconductor laser. 13a. The aiming light Le emitted from the second light source 13 is input to the optical connector 18 via the optical fiber FB8.

The measurement light L1 and the aiming light Le are combined in the optical connector 18, and the combined light is guided to the rotation-side optical fiber FB1 in the optical probe 16.

The optical fiber coupler (the branching/combining section) 14 is composed of a 2x2 optical fiber coupler, for example. The optical fiber coupler 14 is respectively optically connected to the fixed-side optical fiber FB2, the optical fibers FB3, FB5 and FB7.

The optical fiber coupler 14 divides the light La entering from the first light source 12 via the optical fibers FB4 and FB3 into the measurement light (the first light flux) L1 and the reference light L2. The measurement light L1 is caused to enter the fixed-side optical fiber FB2 and the reference light L2 is caused to enter the optical fiber FB5.

The optical fiber coupler 14 further combines the light L2, which enters the optical fiber FB5 and returns through the optical fiber FB5 with the frequency thereof being shifted and the optical path length thereof being changed by the optical path length adjustment section 26 as described later, and the light L3 acquired by the optical probe 16 as described below and guided by the fixed-side optical fiber FB2, and emits the combined light to the optical fibers FB3 (FB6) and FB7.

The optical probe 16 is connected to the fixed-side optical fiber FB2 via the optical connector 18. The measurement light L1 combined with the aiming light Le is caused to enter the rotation-side optical fiber FB1 from the fixed-side optical fiber FB2 via the optical connector 18. The incident measurement light L1 combined with the aiming light Le is transmitted by the rotation-side optical fiber FB1 to illuminate the object to be measured S. The returning light L3 from the object to be measured S is acquired. The acquired returning light L3 is transmitted by the rotation-side optical fiber FB1, and is emitted to the fixed-side optical fiber FB2 via the optical connector 18.

The optical connector 18 combines the measurement light (the first light flux) L1 and the aiming light (the second light flux) Le.

The interference light detection section 20 is connected to the optical fibers FB6 and FB7, and detects as the interference signal the interference light L4 and L5 generated by combining the reference light L2 and the returning light L3 by the optical fiber coupler 14.

Here, the OCT processor 400 includes the detection section 30a which is provided on the optical fiber FB6 branched from the optical fiber coupler 28 and detects the light intensity of the interference light L4, and the detection section 30b which is provided on the optical path of the optical fiber FB7 and detects the light intensity of the interference light L5.

The interference light detection section 20 extracts only an interference amplitude component from the interference light L4 detected from the optical fiber FB6 and the interference light L5 detected from the optical fiber FB7 based on the detection results of the detection sections 30a and 30b.

The processing section 22 detects a region where the optical probe 16 and the object to be measured S contact each other at a measurement position, to be more precise, a region where a surface of a probe outer tube (described later) of the optical probe 16 and the surface of the object to be measured S are regarded to contact each other from the interference signal extracted by the interference light detection section 20. The processing section 22 further acquires the tomographic image from the interference signal detected by the interference light detection section 20, and outputs the acquired tomographic image to the endoscope processor 200.

The optical path length adjustment section 26 is located on the emission side of the reference light L2 of the optical fiber FB5 (that is, an end portion on the opposite side of the optical fiber FB5 from the optical fiber coupler 14).

The optical path length adjustment section 26 includes a first optical lens 80 which shapes the light emitted from the optical fiber FB5 into collimated light, a second optical lens 82 which collects the light shaped into the collimated light by the first optical lens 80, a reflection mirror 84 which reflects the light collected by the second optical lens 82, a base 86 which supports the second optical lens 82 and the reflection mirror 84, and a mirror moving mechanism 88 which moves the base 86 in a direction parallel to an optical axis direction. The optical path length adjustment section 26 adjusts the optical path length of the reference light L2 by changing a distance between the first optical lens 80 and the second optical lens 82.

The first optical lens 80 shapes the reference light L2 emitted from a core of the optical fiber FB5 into the collimated light, and also collects the reference light L2 reflected by the reflection mirror 84 into the core of the optical fiber FB5.

The second optical lens 82 collects the reference light L2 shaped into the collimated light by the first optical lens 80 onto the reflection mirror 84, and also shapes the reference light L2 reflected by the reflection mirror 84 into collimated light. In such a manner, the first optical lens 80 and the second optical lens 82 constitute a confocal optical system.

The reflection mirror 84 is located at the focal point of the light collected by the second optical lens 82, to reflect the reference light L2 collected by the second optical lens 82.

As described above, the reference light L2 emitted from the optical fiber FB5 is shaped into the collimated light by the first optical lens 80, and is collected onto the reflection mirror 84 by the second optical lens 82. Thereafter, the reference light L2 reflected by the reflection mirror 84 is shaped into the collimated light by the second optical lens 82, and is collected into the core of the optical fiber FB5 by the first optical lens 80.

The base 86 fixes the second optical lens 82 and the reflection mirror 84. The mirror moving mechanism 88 moves the base 86 in the optical axis direction (the direction of an arrow A in Fig. 2) of the first optical lens 80.

The distance between the first optical lens 80 and the second optical lens 82 can be changed by moving the base 86 in the direction of the arrow A by the mirror moving mechanism 88. The optical path length of the reference light L2 can be thereby adjusted.

The operation control section 32 includes an input device such as a keyboard and a mouse, and a control device which controls various conditions based on input information. The operation control section 32 is connected to the processing section 22. The operation control section 32 inputs, sets and changes various processing conditions or the like in the processing section 22 based on the instruction of the operator input from the input device.

The operation screen of the operation control section 32 may be displayed on the monitor device 500 or on a display section which is provided separately. The operation control section 32 may also perform operation control and set various conditions of the first light source 12, the second light source 13, the optical connector 18, the interference light detection section 20, the optical path length and the detection sections 30a and 30b.

### [OCT probe]

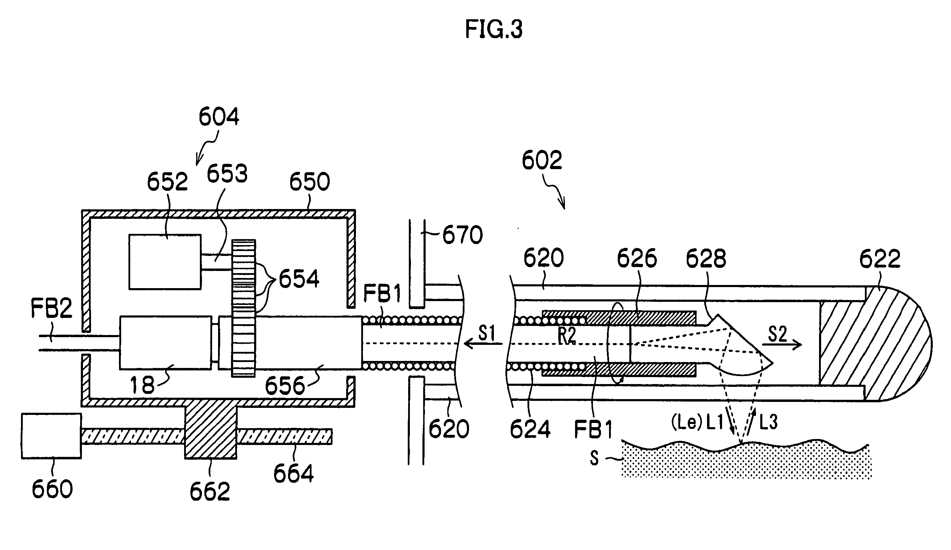

Fig. 3 is a sectional view of the OCT probe 600.

As shown in Fig. 3, the distal end portion of the insertion portion 602 includes a probe outer tube 620, a cap 622, the rotation-side optical fiber FB1, a spring 624, a fixing member 626, and an optical lens 628.

The probe outer tube (sheath) 620 is a tubular member having flexibility, and includes material through which the measurement light L1 combined with the aiming light Le in the optical connector 18 and the returning light L3 are transmitted. As long as a portion of a distal end (a distal end of the rotation-side optical fiber FB1 on the opposite side from the optical connector 18, hereinafter referred to as the distal end of the probe outer tube 620) side of the probe outer tube 620 through which the measurement light L1 (the aiming light Le) and the returning light L3 are transmitted is made of the material through which light is transmitted (transparent material) over the entire periphery, a portion other than the distal end may be made of material through which light is not transmitted.

The cap 622 is provided at the distal end of the probe outer tube 620. The distal end of the probe outer tube 620 is obstructed.

The rotation-side optical fiber FB1 is a linear member, and is housed in the probe outer tube 620 along the probe outer tube 620. The rotation-side optical fiber FB1 guides to the optical lens 628 the measurement light L1 emitted from the fixed-side optical fiber FB2 and combined with the aiming light Le emitted from the optical fiber FB8 in the optical connector 18, and guides to the optical connector 18 the returning light L3 from the object to be measured S acquired by the optical lens 628 by emitting the measurement light L1 (the aiming light Le) onto the object to be measured S, to cause the returning light L3 to enter the fixed-side optical fiber FB2.

Here, the rotation-side optical fiber FB1 and the fixed-side optical fiber FB2 are optically connected to each other by the optical connector 18 in a state in which the rotation of the rotation-side optical fiber FB1 is not transmitted to the fixed-side optical fiber FB2. The rotation-side optical fiber FB1 is disposed in a rotatable manner relative to the probe outer tube 620 and in a movable manner in the axial direction of the probe outer tube 620.

The spring 624 is fixed to the outer periphery of the rotation-side optical fiber FB1. The rotation-side optical fiber FB1 and the spring 624 are connected to the optical connector 18.

The optical lens 628 is located at the distal end of the rotation-side optical fiber FB1 on the measurement side (the distal end of the rotation-side optical fiber FB1 on the opposite side from the optical connector 18). The distal end portion thereof is formed in a substantially spherical shape to collect the measurement light L1 (the aiming light Le) emitted from the rotation-side optical fiber FB1 onto the object to be measured S.

The optical lens 628 illuminates the object to be measured S with the measurement light L1 (the aiming light Le) emitted from the rotation-side optical fiber FB1, and collects the returning light L3 from the object to be measured S to cause the returning light L3 to enter the rotation-side optical fiber FB1.

The fixing member 626 is arranged on the outer periphery of a connection portion between the rotation-side optical fiber FB1 and the optical lens 628. The optical lens 628 is thereby fixed to the end portion of the rotation-side optical fiber FB1. A method of fixing the optical lens 628 to the rotation-side optical fiber FB1 by the fixing member 626 is not specifically limited. The rotation-side optical fiber FB1 and the optical lens 628 may be bonded and fixed to the fixing member 626 by using an adhesive, or may be fixed thereto by a mechanical structure such as a bolt. Any material such as zirconia ferrule and metal ferrule may be used for the fixing member 626 as long as the material is used for fixing, holding or protecting the optical fiber.

The rotation-side optical fiber FB1 and the spring 624 are also connected to a rotating cylinder 656 describe below. The rotating cylinder 656 rotates the rotation-side optical fiber FB1 and the spring 624, thereby rotating the optical lens 628 in the direction of an arrow R2 relative to the probe outer tube 620. The optical connector 18 includes a rotary encoder, and detects the emission position of the measurement light L1 from the position information (angle information) of the optical lens 628 based on a signal from the rotary encoder. That is, the angle of the rotating optical lens 628 in the rotational direction with respect to a reference position is detected to detect the measurement position.

Furthermore, the rotation-side optical fiber FB1, the spring 624, the fixing member 626, and the optical lens 628 are configured to be movable in the directions of an arrow S1 (the direction toward the forceps port) and an arrow S2 (the direction toward the distal end of the probe outer tube 620) inside the probe outer tube 620 by a driving section described below.

Fig. 3 schematically illustrates on its left side the driving section of the rotation-side optical fiber FB1 and the like in the operation portion 604 of the OCT probe 600.

The probe outer tube 620 is fixed to a fixing member 670. Meanwhile, the rotation-side optical fiber FB1 and the spring 624 are connected to the rotating cylinder 656. The rotating cylinder 656 is configured to rotate in accordance with the rotation of a motor 652 via a gear 654 which is mounted on a rotating shaft 653 of the motor 652. The rotating cylinder 656 is connected to the optical connector 18. The measurement light L1 and the returning light L3 are transmitted between the rotation-side optical fiber FB1 and the fixed-side optical fiber FB2 via the optical connector 18.

A frame 650 which incorporates the above components includes a support member 662. The support member 662 includes an unillustrated threaded hole. A ball screw (shaft) 664 for back and forth movement is screwed with the threaded hole. A motor 660 is connected to the ball screw 664 for back and forth movement. Accordingly, by rotationally driving the motor 660, the frame 650 is moved back and forth, so that the rotation-side optical fiber FB1, the spring 624, the fixing member 626 and the optical lens 628 can be moved in the directions of S 1 and S2 in Fig. 3.

The OCT probe 600 has the aforementioned configuration. The rotation-side optical fiber FB1 and the spring 624 are rotated in the direction of the arrow R2 in Fig. 3 by the optical connector 18. The OCT probe 600 thereby illuminates the object to be measured S with the measurement light L1 (the aiming light Le) emitted from the optical lens 628 while performing scanning in the direction of the arrow R2 (the circumferential direction of the probe outer tube 620), and acquires the returning light L3. The aiming light Le is emitted onto the object to be measured S as blue, red or green spot light, for example. The reflected light of the aiming light Le is displayed as a bright spot on an observation image displayed on the monitor device 500.

Accordingly, a desired portion of the object to be measured S can be accurately captured over the entire periphery of the probe outer tube 620 in the circumferential direction, and the returning light L3 reflected at the object to be measured S can be acquired.

In a case of acquiring a plurality of tomographic images for generating the three-dimensional volume data, the optical lens 628 is moved in the direction of the arrow S 1 to one end of a movable range by the driving section. The optical lens 628 then moves in the direction of the arrow S2 by a predetermined distance at a time until reaching the other end of the movable range while acquiring the tomographic images, or alternately acquires the tomographic image and moves a predetermined distance in the direction of S2 until reaching the other end of the movable range.

In such a manner, a plurality of tomographic images are acquired over a desired area of the object to be measured S. The three-dimensional volume data can be obtained based on the acquired plurality of tomographic images.

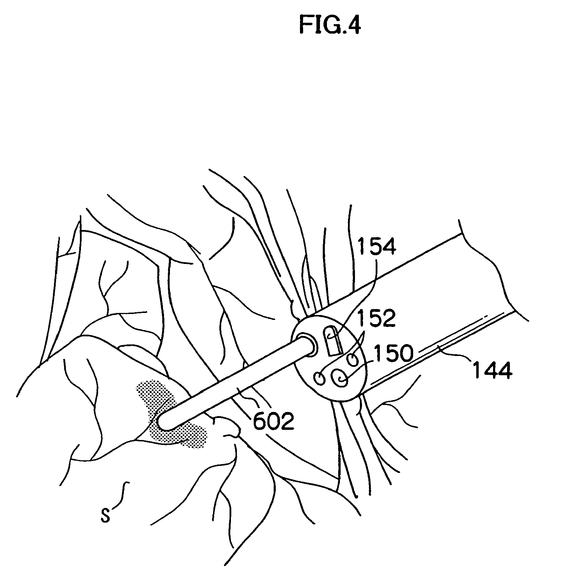

Fig. 4 is a view illustrating a state in which the tomographic image is acquired by using the OCT probe 600 pulled out of the forceps port 156 of the endoscope 100. As shown in Fig. 4, the distal end portion of the insertion portion 602 of the OCT probe is brought closer to a desired portion of the object to be measured S to acquire the tomographic image. When a plurality of tomographic images are acquired over a desired area, the body of the OCT probe 600 does not need to be moved, but the optical lens 628 is moved inside the probe outer tube 620 by the driving section as described above.

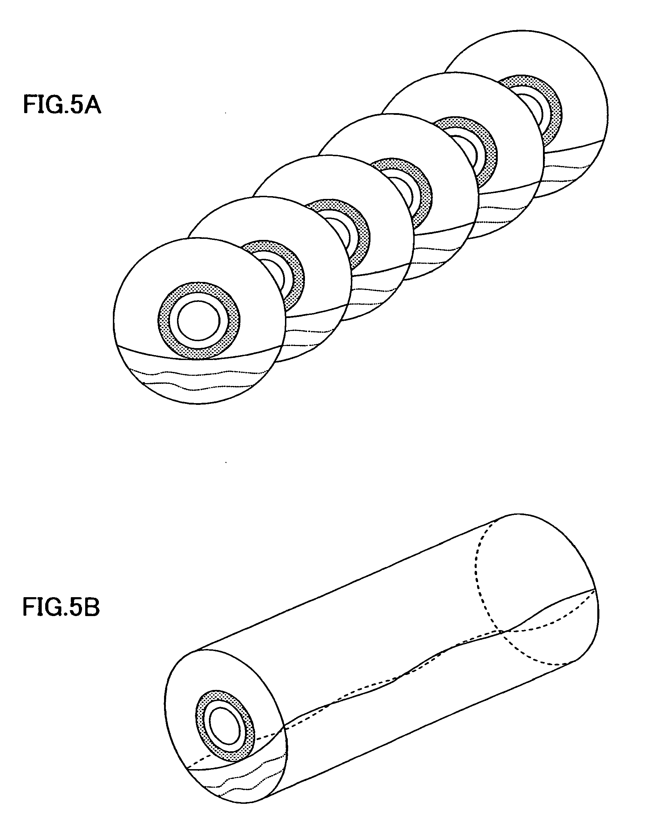

Fig. 5A illustrates the three-dimensional volume data in which the plurality of tomographic images acquired by alternately moving the optical lens 628 of the OCT probe 600 a predetermined distance and acquiring the optical tomographic image are lined up. Fig. 5B illustrates the three-dimensional image generated based on the three-dimensional volume data shown in Fig. 5A. As described above, the three-dimensional volume data can be generated by acquiring the plurality of tomographic images by moving the optical lens 628, and performing image processing thereon.

The three-dimensional image is generated from the three-dimensional volume data on the assumption that the adjacent tomographic images in the volume data are respectively acquired at regular intervals. However, in the mechanism as shown in Fig. 3 which moves the frame 650 back and forth by rotationally driving the motor 660, there may occur operational irregularities such as rotational irregularities of the motor 660, movement irregularities due to machine accuracy, and movement irregularities due to thermal expansion of each component caused by temperature variations, thereby causing the acquisition intervals of the tomographic images to be not regular.

### <Operation of diagnostic imaging apparatus>

The diagnostic imaging apparatus 10 according to the present invention acquires the optical tomographic images by using the probe outer tube 620 on which markers are provided, and performs image processing using the markers appearing in the optical tomographic images, so as to generate the three-dimensional volume data which is not affected by the operational irregularities of the driving section.

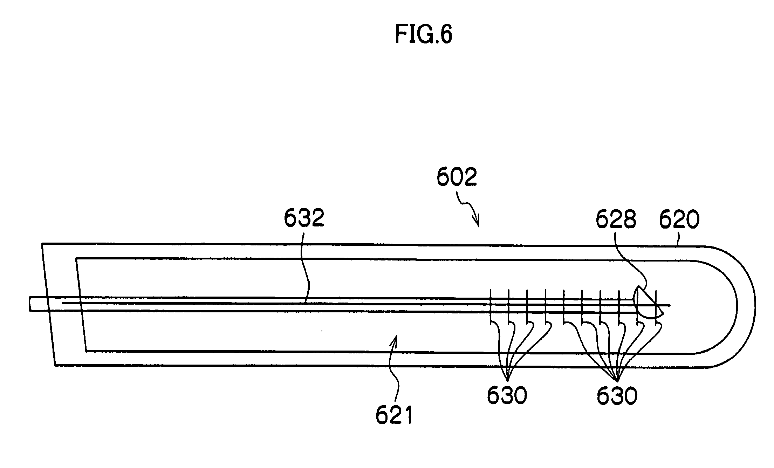

Fig. 6 is a view of the probe outer tube 620 according to the present embodiment. As shown in Fig. 6, the probe outer tube 620 includes a rotational irregularity correction marker 632 which is a line drawn parallel to the long axis direction of the probe outer tube 620, and movement irregularity correction markers 630 which are drawn in a portion of the circumferential direction of the probe outer tube 620 which is perpendicular to the rotational irregularity correction marker 632 at regular intervals in the long axis direction of the probe outer tube 620. The movement irregularity correction markers 630 and the rotational irregularity correction marker 632 are provided on an inner wall surface 621 of the probe outer tube 620.

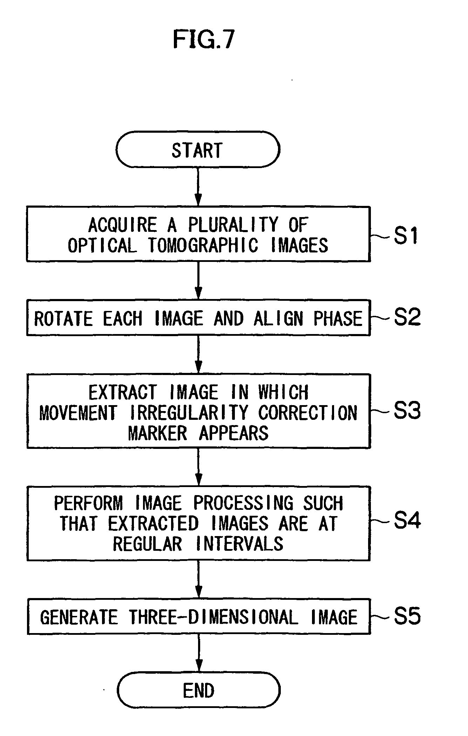

A process of acquiring the three-dimensional volume data by using the probe outer tube 620 with the markers shown in Fig. 6 will be described with reference to Fig. 7.

First, a plurality of optical tomographic images to be used for generating the three-dimensional volume data are acquired (step S1). The operator sets the probe outer tube 620 such that the rotational irregularity correction marker 632 and a certain area of the subject, the tomographic images of which are to be acquired, are 180 degrees opposite from each other on the circumference of the probe outer tube 620. To acquire the plurality of optical tomographic images, the optical lens 628 is moved to the end of the movable range in the direction of the arrow S1 in Fig. 3 by the driving section as described above, and then, alternately acquires the optical tomographic image and moves a predetermined distance in the direction of S2 until reaching the other end of the movable range as described above.

Accordingly, the plurality of optical tomographic images include two types of optical tomographic images: an optical tomographic image acquired at a position where the movement irregularity correction marker 630 exists and an optical tomographic image acquired at a position where the movement irregularity correction marker 630 does not exist (a position where only the rotational irregularity correction marker 632 exists).

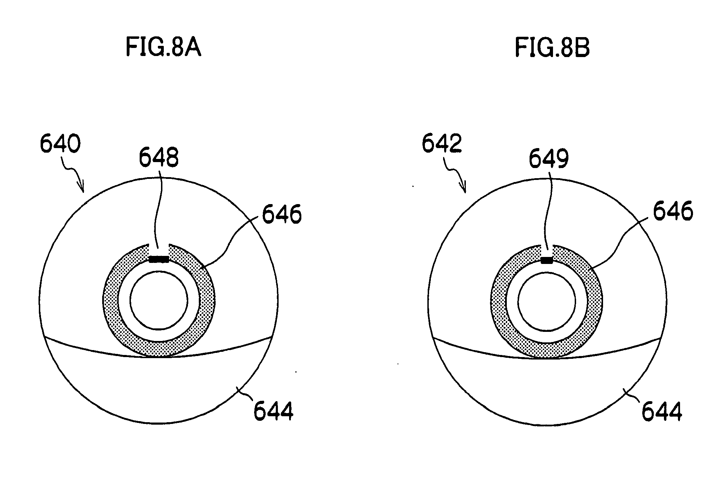

Figs. 8A and 8B are views illustrating the optical tomographic images acquired by using the probe outer tube 620 with the markers shown in Fig. 6. Fig. 8A is a view illustrating an optical tomographic image 640 acquired at a position of the movement irregularity correction marker 630. A tomographic layer 646 of the probe outer tube 620 as well as the tomographic layer of a subject 644 is captured in the optical tomographic image 640. Also, there is a portion where signal light is blocked by the movement irregularity correction marker 630 and no image is extracted, that is, a portion 648 where the movement irregularity correction marker 630 appears in the tomographic layer 646 of the probe outer tube 620.

Fig. 8B is a view illustrating an optical tomographic image 642 acquired at the position where the movement irregularity correction marker 630 does not exist. The tomographic layer 646 of the probe outer tube 620 as well as the tomographic layer of the subject 644 is also captured in the optical tomographic image 642. Also, there is a portion where signal light is blocked by the rotational irregularity correction marker 632 and no image is extracted, that is, a portion 649 where the rotational irregularity correction marker 632 appears in the tomographic layer 646 of the probe outer tube 620. The marker portion 649 has a different length from the marker portion 648 by the movement irregularity correction marker 630.

The plurality of optical tomographic images acquired as described above may be out of phase in the rotational direction of each optical tomographic image due to the rotational irregularities of the motor 652 or the like as described above. The processing section 22 of the OCT processor 400 rotates each image such that the centers of the marker portions 648 and 649 of the respective optical tomographic images are in phase with each other (step S2).

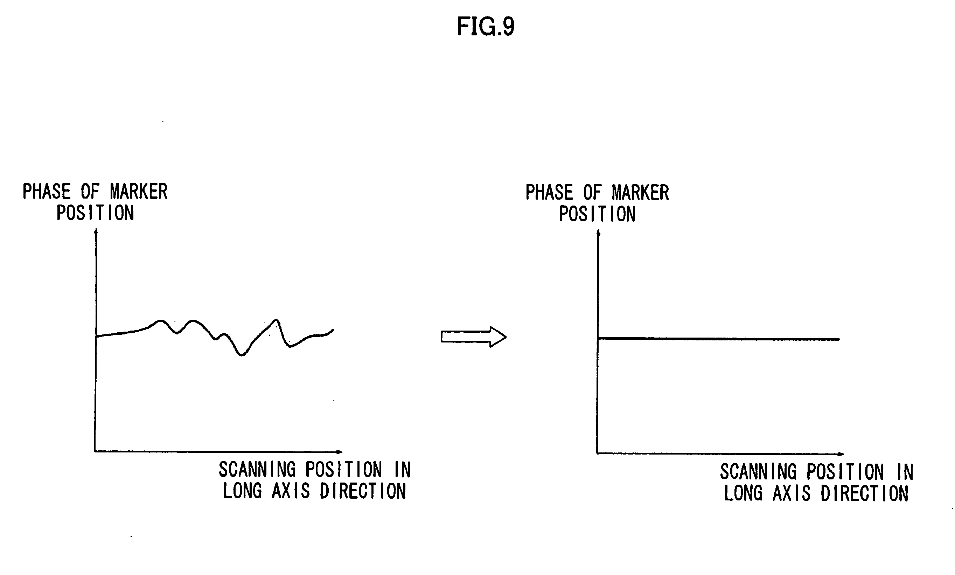

Fig. 9 is a view illustrating that the marker portions 648 are brought in phase with each other by rotating each optical tomographic image. The horizontal axis represents the long axis scanning direction of the probe outer tube 620, and the vertical axis represents the phase of each marker portion 648 and 649. As shown in Fig. 9, even when the images are out of phase in the rotational direction, the images can be brought in phase with each other by rotating each image and aligning the center of each marker portion 648 and 649.

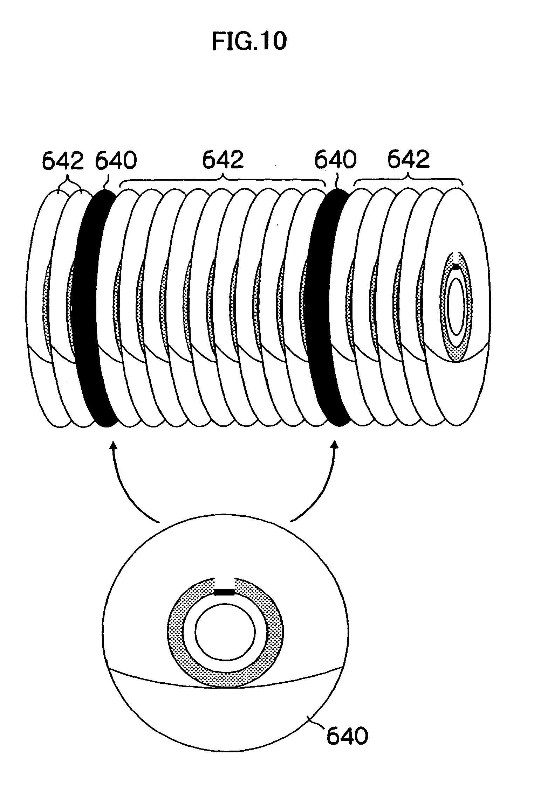

The plurality of optical tomographic images which are brought in phase with each other as described above may differ in interval therebetween due to the rotational irregularities of the motor 660 or the like. To correct the interval, the image 640 in which the marker portion 648 by the movement irregularity correction marker 630 exists is extracted from the plurality of optical tomographic images by the processing section 22 (step S3). Fig. 10 is a view illustrating a state in which the images 640 are extracted. Furthermore, a number of the optical tomographic images 642 acquired at the position where the movement irregularity correction marker 630 does not exist between the extracted images 640 is increased or decreased by the processing section 22 so that the number is constant (step S4). For example, in a case where there are one portion including four images 642 between the images 640 and another portion including five images 642 between the images 640, one of the five images 642 may be subtracted or two of the five images 642 may be averaged to one image to even out the number of the optical tomographic images.

The three-dimensional image is generated by using the plurality of optical tomographic images on which the aforementioned process is performed as the volume data (step S5). Accordingly, a phase accuracy and position accuracy of the volume data can be improved.

There may be no rotational irregularity correction marker 632 on the probe outer tube 620. In this case, each image 640 is rotated such that the centers of the marker portions 648 are in phase with each other, and the images 642 therebetween may be brought in phase with each other by using a rotation amount predicted from the rotation amount of each image 640.

### <Thickness of movement irregularity correction marker>

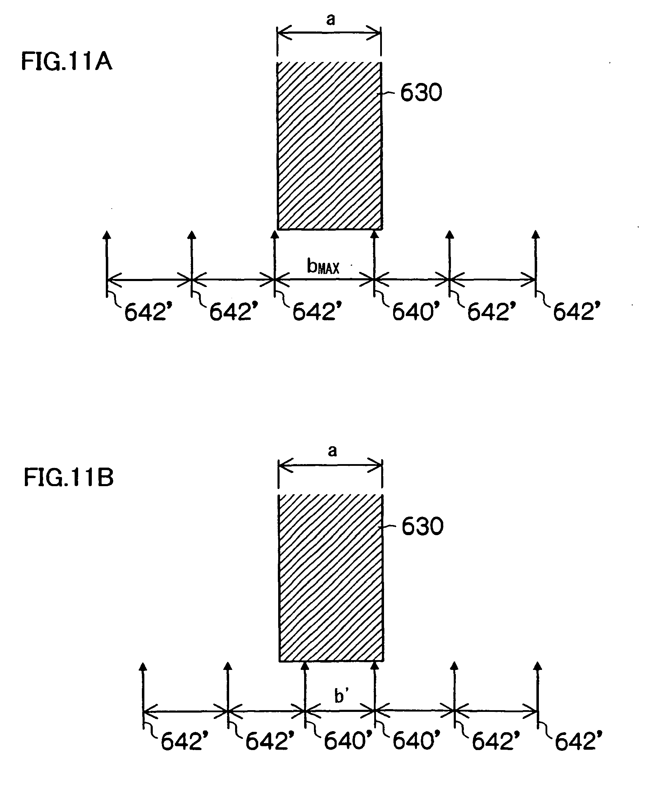

Figs. 11A and 11B are views illustrating an example of the movement irregularity correction marker 630 and the acquisition positions of the images 640 and 642. Reference numeral 640' designates the acquisition position of the image 640, and reference numeral 642' designates the acquisition position of the image 642. As shown in Fig. 11A, thickness a of the movement irregularity correction marker 630 is preferably thicker than a maximum value bₘₐₓ of a distance between the acquisition positions of the optical tomographic images in consideration of the operational irregularities of the driving section. With such thickness, the optical tomographic images can be prevented from being acquired skipping the movement irregularity correction marker 630. That is, the images 640 can be acquired for all the movement irregularity correction markers 630.

When the thickness a of the movement irregularity correction marker 630 is thicker than bₘₐₓ. two consecutive optical tomographic images may be acquired at the position of the movement irregularity correction marker 630 as shown in Fig. 11B. In a case where two consecutive images 640 are acquired as described above, the volume data may be generated by recognizing that the movement irregularity correction markers 630 therein are the same marker 630 and there exists no image 642 therebetween.

## Claims

1. A three-dimensional image acquisition apparatus (10), comprising:
an optical probe (600) adapted to be inserted through a forceps port (156) of an endoscope (100) into a living body as an object to be measured, said optical probe comprising:
a sheath (620) whose distal end portion at least is optically transparent to signal light;
an optical fiber (FB1) configured to guide the signal light, the optical fiber being arranged inside the sheath;
a lens (628) configured to emit the signal light from a side surface of the distal end portion of the sheath and into which returning light from the living body enters, the lens being coupled to a distal end of the optical fiber;
a flexibly shaft (624) configured to transmit torque to rotate the optical fiber and the lens and to transmit driving power to move the optical fiber and the lens back and forth; said apparatus further comprising a driving device (652, 660) configured to transmit torque and driving power to the flexible shaft, the driving device being coupled to the optical probe;
an optical tomographic image generation device (20, 22) configured to generate an optical tomographic image based on a returning light returned from the living body obtained by the optical probe and to generate optical tomographic images, a number of the optical tomographic images corresponding to the number of rotations of the flexible shaft; **characterised in that** said optical probe further comprises
a plurality of first markers (630) which are provided at predetermined intervals on the distal end portion of the sheath along a longitudinal direction of the sheath and which can be read by said lens (628); and that said apparatus further comprises
a first marker extraction device (22) configured to extract first markers from the plurality of optical tomographic images; and
an adjustment device (22) configured to adjust the number of optical tomographic images such that the number of optical tomographic images between the optical tomographic images from which the first markers are extracted is equal to each other.

2. The three-dimensional image acquisition apparatus (10) according to claim 1, wherein a second marker (632) which is a straight line parallel to the longitudinal direction of the sheath is provided on the distal end portion of the sheath;
further comprising:
a second marker extraction device (22) for extracting second markers from the plurality of optical tomographic images; and
a rotating device (22) for rotating the plurality of optical tomographic images such that the extracted second markers are aligned with each other.

3. The apparatus according to claim 1, wherein a second marker (632) which is a straight line parallel to the longitudinal direction of the sheath is provided on the distal end portion of the sheath.

4. The apparatus according to claim 2 or 3, wherein the second marker is provided on an inner wall surface (621) of the distal end portion of the sheath.

5. The apparatus according to claim 2, 3 or 4, wherein the first markers and the second marker are provided perpendicular to each other and which can both be read by said lens (628).

6. The apparatus according to any of claims 1 to 5, wherein the first markers are provided on an inner wall surface (621) of the distal end portion of the sheath.

7. The three-dimensional image acquisition apparatus according to claim 1 or 2, further comprising a generation device (22) for generating a three-dimensional image based on the plurality of optical tomographic images.

## Patentansprüche

1. Erfassungsvorrichtung (10) für dreidimensionale Bilder, umfassend:
eine optische Sonde (600), ausgebildet zum Einführen durch eine Zangenöffnung (156) eines Endoskops (100) in einen lebenden Körper als Messobjekt, wobei die optische Sonde aufweist:
eine Hülle (620), deren distaler Endabschnitt zumindest für Signallicht optisch transparent ist;
eine optische Faser (FB1), konfiguriert zum Führen des Signallichts, wobei die optische Faser im Inneren der Hülle angeordnet ist;
ein Objektiv (628), konfiguriert zum Emittieren des Signallichts von einer Seitenfläche des distalen Endabschnitts der Hülle, und in welches Rücklicht von dem lebenden Körper eintritt, wobei das Objektiv an ein distales Ende der optischen Faser gekoppelt ist;
einen flexiblen Schaft (624), konfiguriert zum Übertragen von Drehmoment zum Drehen der optischen Faser und des Objektivs und zum Übertragen von Antriebsleistung zum Bewegen der optischen Faser und des Objektivs zurück und nach vorn; wobei die Vorrichtung außerdem aufweist:
eine Antriebseinrichtung (652, 660), konfiguriert zum Übertragen von Drehmoment und Antriebsleistung auf den flexiblen Schaft, wobei die Antriebseinrichtung mit der optischen Sonde gekoppelt ist;
eine optische Tomographiebild-Erzeugungseinrichtung (20, 22), konfiguriert zum Erzeugen eines optischen Tomographiebilds basierend auf einem von dem lebenden Körper zurückkommenden Rücklicht, welches von der optischen Sonde gewonnen wird, und zum Erzeugen optischer Tomographiebilder, wobei eine Anzahl der optischen Tomographiebilder der Anzahl von Umdrehungen des flexiblen Schafts entspricht, **dadurch gekennzeichnet, dass** die optische Sonde außerdem aufweist:
eine Mehrzahl erster Markierungen (630), die an vorbestimmten Intervallen an dem distalen Endabschnitt der Hülle in Längsrichtung der Hülle vorgesehen sind, und die von dem Objektiv (628) gelesen werden können; wobei die Vorrichtung außerdem aufweist:
eine erste Markierungs-Extraktionseinrichtung (22), konfiguriert zum Extrahieren erster Markierungen aus den mehreren optischen Tomographiebildern; und
eine Justiereinrichtung (22), konfiguriert zum Justieren der Anzahl optischer Tomographiebilder in der Weise, dass die Anzahl optischer Tomographiebilder zwischen den optischen Tomographiebildern, aus denen die ersten Markierungen extrahiert wurden, jeweils gleich groß ist.

2. Erfassungsvorrichtung (10) für dreidimensionale Bilder nach Anspruch 1, bei der eine zweite Markierung (632), bei der es sich um eine gerade Linie parallel zur Längsrichtung der Hülle handelt, an dem distalen Endabschnitt der Hülle vorgesehen ist, weiterhin umfassend:
eine zweite Markierungs-Extraktionseinrichtung (22) zum Extrahieren zweiter Markierungen aus den mehreren optischen Tomographiebildern; und
eine Dreheinrichtung (22) zum Drehen der mehreren optischen Tomographiebilder derart, dass die extrahierten weiten Markierungen miteinander fluchten.

3. Vorrichtung nach Anspruch 1, bei der eine zweite Markierung (632), bei der es sich um eine gerade Linie parallel zur Längsrichtung der Hülle handelt, an dem distalen Endabschnitt der Hülle vorgesehen ist.

4. Vorrichtung nach Anspruch 2 oder 3, bei der die zweite Markierung an der Innenwandfläche (621) des distalen Endabschnitts der Hülle vorgesehen ist.

5. Vorrichtung nach Anspruch 2, 3 oder 4, bei der die ersten Markierungen und die zweiten Markierung rechtwinklig zueinander angeordnet sind, und beide von dem Objektiv (628) gelesen werden können.

6. Vorrichtung nach einem der Ansprüche 1 bis 5, bei der die ersten Markierungen an einer Innenwandfläche (621) des distalen Endabschnitts der Hülle vorgesehen sind.

7. Erfassungsvorrichtung für dreidimensionale Bilder nach Anspruch 1 oder 2, weiterhin umfassend eine Erzeugungseinrichtung (22) zum Erzeugen eines dreidimensionalen Bilds basierend auf den mehreren optischen Tomographiebildern.

## Revendications

1. Appareil d'acquisition d'images tridimensionnelles (10), comportant :
une sonde optique (600) adaptée pour être insérée par l'intermédiaire d'un orifice de forceps (156) d'un endoscope (100) dans un être vivant en tant qu'objet à mesurer, ladite sonde optique comportant :
un manchon (620) dont la partie terminale distale est au moins optiquement transparente à une lumière de signal ;
une fibre optique (FB1) configurée pour guider la lumière de signal, la fibre optique étant agencée à l'intérieur du manchon ;
une lentille (628) configurée pour émettre la lumière de signal depuis une surface latérale de la partie terminale distale du manchon et dans laquelle une lumière provenant de l'être vivant pénètre, la lentille étant couplée à une extrémité distale de la fibre optique ;
un arbre flexible (624) configuré pour transmettre un couple pour faire pivoter la fibre optique et la lentille et pour transmettre une puissance d'entraînement pour déplacer la fibre optique et la lentille dans un mouvement de va-et-vient ; ledit appareil comportant en outre :
un dispositif d'entraînement (652, 660) configuré pour transmettre un couple et une énergie d'entraînement à l'arbre flexible, le dispositif d'entraînement étant couplé à la sonde optique ;
un dispositif de génération d'images tomographiques optiques (20, 22) configuré pour générer une image tomographique optique en fonction d'une lumière de retour renvoyée depuis l'être vivant obtenue par la sonde optique et pour générer des images tomographiques optiques, un nombre d'images tomographiques optiques correspondant au nombre de rotations de l'arbre flexible ; **caractérisé en ce que** ladite sonde optique comporte en outre
une pluralité de premiers marqueurs (630) qui sont prévus à des intervalles prédéterminés sur la partie terminale distale du manchon le long d'une direction longitudinale du manchon et qui peuvent être lus par ladite lentille (628) ; et **en ce que** ledit appareil comporte en outre
un dispositif d'extraction de premiers marqueurs (22) configuré pour extraire les premiers marqueurs de la pluralité d'images tomographiques optiques ; et
un dispositif de réglage (22) configuré pour régler le nombre d'images tomographiques optiques de sorte que le nombre d'images tomographiques optiques entre les images tomographiques optiques à partir desquelles sont extraits les premiers marqueurs est égal à l'autre.

2. Appareil d'acquisition d'images tridimensionnelles (10) selon la revendication 1, dans lequel un second marqueur (632) qui est une ligne droite parallèle à la direction longitudinale du manchon est prévu sur la partie terminale distale du manchon ;
comportant en outre :
un dispositif d'extraction de seconds marqueurs (22) pour extraire des seconds marqueurs à partir de la pluralité d'images tomographiques optiques ; et
un dispositif pivotant (22) pour faire pivoter la pluralité d'images tomographiques optiques de sorte que les seconds marqueurs extraits sont alignés les uns avec les autres.

3. Appareil selon la revendication 1, dans lequel un second marqueur (632) qui est une ligne droite parallèle à la direction longitudinale du manchon est prévu sur la partie terminale distale du manchon.

4. Appareil selon la revendication 2 ou 3, dans lequel le second marqueur est prévu sur une surface de la paroi interne (621) de la partie terminale distale du manchon.

5. Appareil selon la revendication 2, 3 ou 4, dans lequel les premiers marqueurs et le second marqueur sont prévus perpendiculaires les uns aux autres et qui peuvent tous être lus par ladite lentille (628).

6. Appareil selon l'une quelconque des revendications 1 à 5, dans lequel les premiers marqueurs sont prévus sur une surface de la paroi interne (621) de la partie terminale distale du manchon.

7. Appareil d'acquisition d'images tridimensionnelles selon la revendication 1 ou 2, comportant en outre un dispositif de génération (22) pour générer une image tridimensionnelle en fonction de la pluralité d'images tomographiques optiques.
